# EUROPEAN PATENT SPECIFICATION

(11) **EP 2 555 687 B1**
(45) Date of publication and mention of the grant of the patent: **04.11.2015**
(21) Application number: 11713968.3
(22) Date of filing: 04.04.2011
(51) Int. Cl.: A61B 10/02, A61B 10/04

(54) **ENDOSCOPIC ULTRASOUND-GUIDED BIOPSY NEEDLE**
ENDOSKOPISCHE ULTRASCHALLGEFÜHRTE BIOPSIENADEL
AIGUILLE ENDOSCOPIQUE À BIOPSIE GUIDÉE PAR ULTRASONS

(30) Priority: 06.04.2010 US 321243 P
(43) Date of publication of application: 13.02.2013
(60) Divisional application: 15185299.3
(73) Proprietor: Cook Medical Technologies LLC, Bloomington, IN 47404 (US)
(72) Inventor: SCHEMBRE, Drew, B., Washington 98109 (US); CLANCY, Michael, S., Monaleen, Limerick (IE); CHMURA, Kevin, Lewisville, NC 27023 (US)
(74) Representative: Garratt, Peter Douglas
(86) International application number: PCT/US2011/031048
(87) International publication number: WO 2011/126963

(56) References cited:
- WO-A1-03/079907
- US-A- 5 199 441
- US-A- 5 320 110
- US-A1- 2004 249 288
- US-A1- 2005 090 765
- US-A1- 2005 101 879

## Description

### TECHNICAL FIELD

The invention relates generally to endoscopic surgical devices. More particularly, the invention pertains to a biopsy needle configured for use during minimally-invasive procedures such as endoscopic procedures.

### BACKGROUND

Fine needle aspiration (FNA) is a diagnostic biopsy procedure used to obtain a sample from a target site in a patient body. A fine needle (e.g., 19-gauge to 25-gauge) is directed to a target site, and suction is applied to the proximal end of a lumen of the needle to aspirate cells through its distal end. The procedure typically is far less invasive than other biopsy techniques, whether performed percutaneously (e.g., to sample a suspected breast tumor or subcutaneous lesion) or endoscopically (e.g., to sample a suspected cholangiocarcinoma via a duodenoscope). Moreover, advances in endoscopic ultrasound (EUS) technology have helped physicians and patients by providing enhanced ability of a physician to visualize a biopsy needle to obtain a sample of material from a target site without requiring an open incision or use of large-bore needles and/or laparoscopic trocars.

Current FNA techniques typically obtain only a small number of cells useful for diagnostic evaluation. As a result, this technique includes a risk of false negatives where the few cells obtained in a sample do not accurately represent the presence of a tumor or other disease condition. The small sample size may also limit the diagnostic value of the procedure if the cells obtained are sufficiently few in number or sufficiently damaged during collection that they do not enable a definitive diagnosis. Accordingly it would be advantageous to provide a needle useful for EUS and/or percutaneous FNB (fine needle biopsy) that can obtain a larger sample size (e.g., a larger number of cells in the sample or a "core" comprising intact adjacent cells held together in similar form to their native location) without requiring a larger-gauge needle or requiring multiple passes of the needle to reliably obtain a diagnostically efficacious sample with regard to the number and integrity of the cells in the sample.
Reference is directed to US 5,199,441 which discloses fine needle aspiration biopsy apparatus in which fenestrations are coated adjacent the sharpener tip of the hollow needle.

The pre-amble of claim 1 is based on this document. Furthermore, US 2005/090765 discloses a biopsy needle having a notch with a distal lip. US 2004/249288 shows a biopsy needle having echogenic surface features.

### BRIEF SUMMARY

Embodiments of needles disclosed here address these problems of the current technology and present advantages over existing needles with regard to structure. A tissue-sampling needle device according to the present invention is defined in claim 1.

### BRIEF DESCRIPTION OF THE DRAWINGS

In order to assist the understanding of embodiments of the invention, reference will now be made to the appended drawings, which are not necessarily drawn to scale or proportion, and in which like reference numerals generally refer to like elements. The drawings are exemplary only, and should not be construed as limiting the invention.
FIGS. 1A-1D show different views of a non-claimed tissue-sampling needle device embodiment;
FIGS. 2A-2B show two views of another tissue-sampling needle device embodiment according to the present invention;
FIG. 3 shows another tissue-sampling needle device embodiment according to the present invention; and
FIGS. 4A-4C show a non-claimed method of using a tissue-sampling needle device embodiment.

### DETAILED DESCRIPTION

As used herein, the term "proximal" refers to the handle-end of a device held by a user, and the term "distal" refers to the opposite end. The term "surgical visualization device" refers to endoscopes including CCD, ultrasound, fiber optic, and CMOS devices, as well as other devices used for visualizing an internal portion of a patient body such as, for example, a laparoscope or bronchoscope.

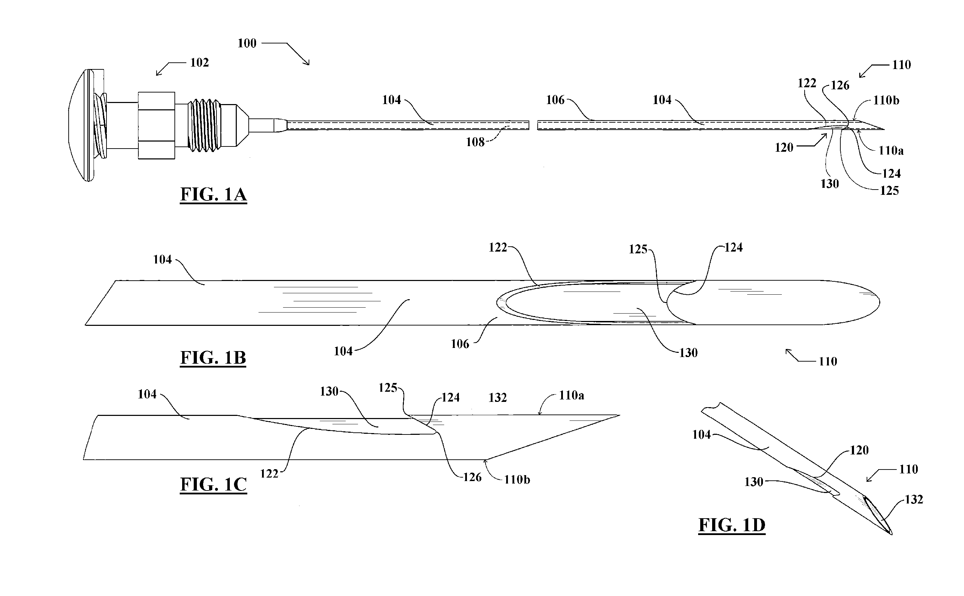

One non-claimed embodiment of a tissue-sampling needle device is described with reference to FIGS. 1A-1D, which show a tissue-sampling needle device 100. As shown in the side plan view of FIG. 1A, the device includes a proximal handle or hub 102 from which an elongate tubular cannula 104 extends distally. The cannula 104 includes a cannula wall 106 that defines a cannula lumen 108. A distal end 110 of the cannula 104 is beveled, including a long side 110a substantially parallel with the central longitudinal axis of the cannula 104 and extending to its distal-most tip end. A short side 110b of the beveled distal end 110 is opposite the long end 110a. A detailed illustration of the distal end 110 is shown in the top plan view of FIG. 1B. Other embodiments may include a double bevel, where one beveled surface is opposite the notch, or single or double bevels that are at least partially transverse relative to the notch.

As shown in the side plan view of FIGS. 1A and 1C, and in the perspective view of FIG. 1D, a notch 120 is disposed proximally adjacent to the beveled distal cannula end 110 and is generally centered in longitudinal alignment with the long beveled end side 110a and opposite the short beveled end side 110b. In preferred embodiments, the notch 120 is generally arcuate, defined on its proximal side by a parabolic edge 122 extending along generally longitudinal, but somewhat curved lateral notch sides 124. The distal edge 124 of the notch 120 preferably is formed as generally parabolic lip that joins the proximal edge 122 at a pair of lip end portions 126 that preferably provide a curved transition between the proximal lateral and distal edges 122, 124. The radiused lip end portions 126 preferably are configured to provide stress relief within the cannula structure. A central distal lip portion 125 of the distal edge 124 preferably forms a proximal-facing cutting edge. In preferred embodiments, the notch will occupy about one-half the circumference of the cannula 104 at the broadest point of the notch. Inclusion of the bevel illustrated in FIG. 1C may provide advantages for successful sample collection. Specifically, contact of the bevel face against tissue may create a slight bias/ pressure toward the notch that will help tissue to be pulled/ captured into the notch when the stylet is withdrawn, including that contact pressure on the notch side of the shaft surface may be slightly greater than on the exterior surface immediately opposite the notch.

An elongate stylet 130 may be disposed removably through the cannula lumen 108. In preferred embodiments the stylet 130 will occupy substantially an entire cross-sectional area of at least a lengthwise portion of the cannula lumen 108. And, as shown in FIG. 1D, a distal end 132 of the stylet 130 will be beveled to align with the distal beveled cannula end 110 in a substantially coplanar manner. This construction will provide enhanced support for the cannula (particularly during navigation to a target site) including providing a generally solid cutting and/or tissue-penetrating distal tip end formed by the matching bevels of the stylet 130 and cannula 104. It should be appreciated that a rounded stylet end or other stylet end configuration may be practiced within the scope of the present invention. In certain embodiments, the distal beveled needle end may be closed, such that the lumen 108 extending longitudinally through the cannula terminates within the cannula 104. In these embodiments, a stylet may be reinserted into the needle lumen after the sample has been excised and captured through the notch into the needle lumen. In such a circumstance, the stylet may be extended distally to cover the open notch

(thereby preventing contamination of the sample by inadvertent collection of cells along the needle track during withdrawal of the needle), but leaving room in a closed needle lumen portion for the sample to remain intact between the notch and a closed distal end in an embodiment where the needle lumen is closed at the distal end.

In one exemplary embodiment, the cannula 104 may be constructed as a 19-gauge needle made of 304 stainless steel, with an inner diameter of about 0.9 mm (about 0.037 inches). In this embodiment, the notch 120 may be circumferentially located opposite and proximal of a distal bevel that is at about a 30° angle relative to the short side such that a proximal-most end of the notch 120 (defined by the proximal edge 122) is about 9 mm (about 0.36 inches) longitudinally proximal of the distal-most tip end of the cannula 104. In this embodiment, the longitudinal distance between the proximal-most notch edge 122 and the proximal-most portion of the distal lip 125 may be about 4 mm to about 5 mm (about 0.16 to about 0.2 inches). The proximal-most portion of the distal lip 125 may be about 0.6 mm (about 0.025 inches) from the distal-most end of the notch 120, which will be defined by a curved lip end portion 126, including a radius of curvature of about 0.05 mm (0.002 inches), joining the distal edge 124 with the proximal edge 122. The longitudinal linear distance between the distal-most lip end portions 126 and the proximal end of the bevel 110 opposite the notch 120 may be about 1.47 mm (0.058 inches) in 19-gauge, 20-gauge, 21-gauge, or 22-gauge embodiments to provide optimal strength and notch position relative to the needle's distal end. The 19-gauge needle may be used with a sheath measuring about 4.2 to 5.2 Fr. A beveled NiTi stylet 130 may be disposed slidably/ removably through the cannula lumen. It should be appreciated that, while a needle not larger than a 19-gauge needle is preferred, smaller gauge needles such as - for example - 22-gauge and 25-gauge needles may be practiced within the scope of the present invention (although, it will be appreciated that their absolute dimensions will vary from those disclosed here for the 19-gauge example).

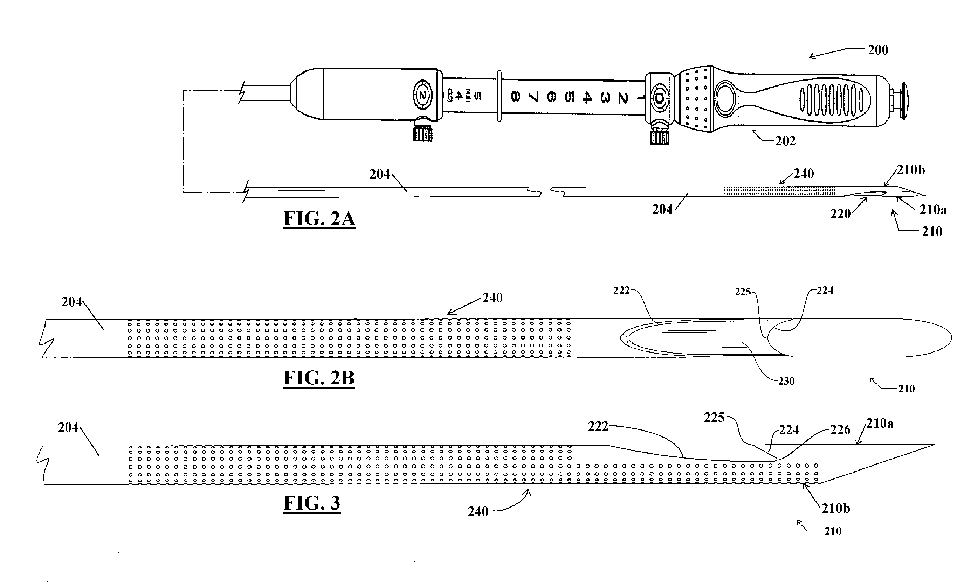

FIGS. 2A, 2B and 3 show embodiments of a tissue-sampling needle device 200. As shown in the side plan view of FIG. 2A, the device includes a proximal handle or hub 202 from which an elongate tubular cannula 204 extends distally. The cannula 204 includes a cannula wall that defines a cannula lumen. A distal end 210 of the cannula 204 is beveled, including a long side 210a substantially parallel with the central longitudinal axis of the cannula 204 and extending to its distal-most tip end. A short side 210b of the beveled distal end 210 is opposite the long end 210a. A detail view of the needle device 200 is shown in a top plan view in FIG. 2B. The distal end 210 may be open to the lumen or may be closed. In embodiments with an open end 210, a sample may be ejected out the distal end after collection.

As shown in the side views of FIGS. 2A and 3, a notch 220 is disposed proximally adjacent to the beveled distal cannula end 210 and is generally centered in longitudinal alignment with the long beveled end side 210a and opposite the short beveled end side 210b. In preferred embodiments, the notch 220 is generally arcuate, defined on its proximal side by a parabolic edge 222 extending along generally longitudinal, but somewhat curved lateral notch sides 224. The distal edge 224 of the notch 220 preferably is formed as generally parabolic lip that joins the proximal edge 222 at a pair of lip end portions 226 that preferably provide a curved transition between the proximal and distal edges 222, 224. A central distal lip portion 225 of the distal edge 224 preferably forms a proximal-facing cutting edge. In preferred embodiments, the notch will occupy about one-half the circumference of the cannula 204 at the broadest point of the notch.

As shown in FIG. 2A, the cannula 204 includes surface features 240 configured to enhance echogenicity, thereby providing an improved ability to navigate the device during an EUS procedure. The surface features 240 are shown here as dimples on an exterior surface of the cannula 204, but may alternatively be embodied as grooves or other regular or irregular features on an external or internal surface. Embedded echogenic features such as bubbles, voids, or pieces of echo-contrasting materials may also be used within the scope of the present invention. Those of skill in the art will appreciate that many currently-known and/or future-developed echogenicity-enhancing means may be used within the scope of the present invention. As used herein, the terms echogenic and echogenicity-enhancing are used to refer to structural features that increase the reflectivity of ultrasound waves used during ultrasound visualization of a device, with the increase being over the typical ultrasound reflectivity/ visualizability of a device lacking the features described.

FIG. 3 is similar to FIG. 2A, but shows that the echogenic features 240 may extend distally across the space occupied by the notch 220. It is preferable that echogenicity-enhancing features be disposed at a specified predetermined distance from the distal-most tip end of the cannula 204. Although the echogenic features 240 are shown at a distance from the notch 220, a cannula according to the present embodiments may be constructed with those echogenic features disposed flush up to the margins of the notch. A stylet 230, which may include echogenicity-enhancing features, may be disposed through the cannula lumen of the embodiments of FIGS. 2A-3.

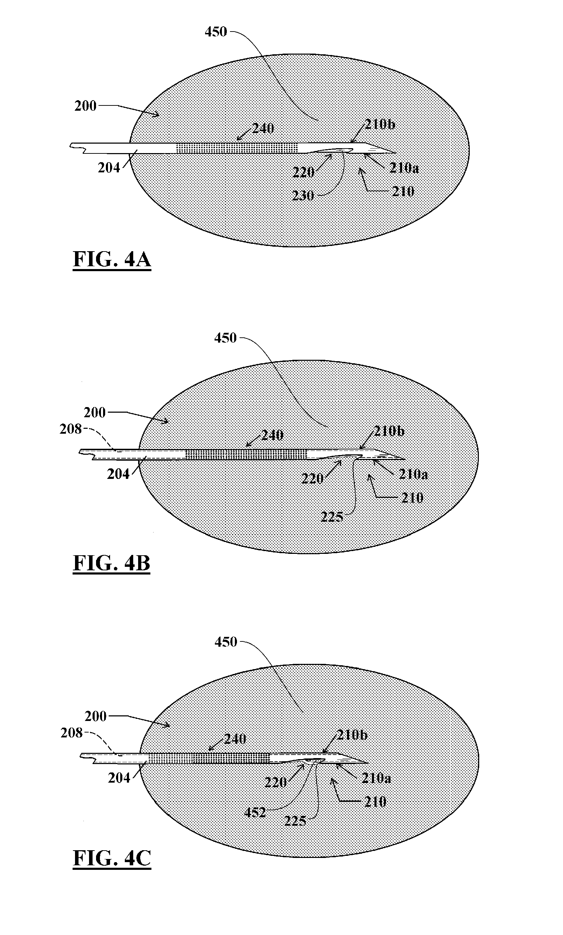

A non-claimed method of tissue collection is described with reference to FIGS. 4A-4C, using the needle device 200 of FIGS. 2A-2B. First, as shown in FIG. 4A, the needle cannula 204, with the stylet 230 disposed therein, is directed into a target site 450 to be sampled (e.g., a tumor mass). Next, as shown in FIG. 4B, the stylet 230 is withdrawn and suction is applied to the proximal end of the needle cannula lumen 208. This will pull tissue from the target site 450 through the notch 220 into the lumen 208. As shown in FIG. 4C, the user will then quickly retract the cannula 204 proximally such that the proximal-facing cutting edge of the distal notch's central lip 225 cuts a sample 452 of tissue from the target site that is drawn into the lumen 208 and that may be captured within, distal, or proximal of the notch 220. The cannula 204 may be advanced and retracted slightly (e.g., about half a centimeter, two or three times) and/or rotated or otherwise manipulated by the user if desired to try to capture sample material. The cannula 204 may be bowed slightly during use to accentuate contact of the notch 220 with adjacent tissue to promote improved sample collection.

The sample obtained preferably will include a desirable number of intact cells, preferably more intact cells than are ordinarily obtained using a non-notched FNA biopsy needle ("more" indicating both a greater number and a higher degree of cell integrity within the sample obtained). It has been found that histological-grade FNB samples may be obtained in this manner, which may be preferred for certain diagnostic purposes over the cytological-grade samples typically obtained through FNA. The needle may then be withdrawn from the patient's body.

In one preferred embodiment, during introduction of the device into a patient body, the cannula 204 will be directed through the working channel of a peroral endoscope such as a duodenoscope into a patient's body. It is then navigated (under ultrasound visualization if echogenicity-enhancing features are provided, as in the embodiment shown in FIGS. 2A-3) into the target site 450. In other embodiments, the device 200 may be introduced through other access means known in the art including percutaneous means such as direct insertion of the needle cannula through a patient's skin or insertion through a trocar, sheath, or other access device (with or without endoscopic or ultrasound visualization), all within the scope of the present invention. It should also be appreciated that an outer sheath may be disposed slidably along the exterior of the cannula 204 and advanced over the notch 220 after the sample is excised by the cutting edge. This configuration, which may be practiced within the scope of the present invention, may lessen the likelihood that the sample collected will become lost or contaminated during needle withdrawal.

The needle device and methods disclosed here provide the advantages associated with FNA needles of small size and maneuverability, while offering a means of collecting more intact samples from target sites. They also are not hampered by the guillotine-style moving parts of other notched needle systems known in the biopsy art (which are generally larger in scale due to a need for having a cutting member that movably transects the notch).

## Claims

1. A tissue-sampling needle device (200), comprising:
an elongate tubular cannula (204) including a cannula wall defining a cannula lumen;
wherein the cannula lumen extends longitudinally through the cannula;
a distal beveled end (210) of the cannula including a long side (210a) and a short side (210b);
a notch (220) through the cannula wall, open to the cannula lumen;
wherein the notch is disposed proximally adjacent to the beveled distal cannula end and is generally centered in longitudinal alignment with the long beveled end side and opposite the short beveled end side;
**characterised in that** the notch includes a distal lip (224) defined by a portion of the cannula wall, the distal lip configured to extend proximally from a distal-most end of the notch such that a central distal lip portion is disposed proximal of lip end portions that are continuous with generally longitudinal lateral sides of the notch; and
wherein the distal lip includes a proximal-facing cutting edge (225);
further comprising a pattern of echogenic surface features (240) disposed immediately adjacent to and proximal of the notch.

2. The device of claim 1, further comprising a stylet disposed through, and occupying substantially an entire cross-sectional area of, at least a lengthwise portion of the cannula lumen, wherein a distal end of the stylet is beveled to align with the distal beveled cannula end in a substantially coplanar manner.

3. The device of claim 1, wherein the cannula comprises a 19-gauge needle including an inner diameter of about 0.9 mm (about 0.037 inches), wherein the notch occupies about one-half of an outer circumference of the cannula.

4. The device of claim 3, wherein a circumferentially central longitudinal length of the notch from the proximal notch end to the central distal lip portion is about 4 mm (about 0.16 inches).

5. The device of claim 4, wherein the central distal lip is disposed about 0.6 mm (about 0.025 inches) longitudinally from a distal-most end of the notch.

6. The device of claim 5, wherein a transitional curving surface between the distal lip and the generally longitudinal lateral sides of the notch comprises a radius of curvature of about 0.05 mm (0.002 inches).

7. The device of claim 1, wherein the echogenic surface features comprise dimples in the cannula wall.

8. The device of claim 1, wherein the cannula is configured as sufficiently flexible for passage through a working channel of a peroral surgical visualization device.

9. The device of claim 1, wherein the cannula comprises a selected one of a 19-gauge needle, 22-gauge needle, or 25-gauge needle, wherein the notch occupies about one-half of an outer circumference of the cannula.

10. The device of claim 1, wherein the lip end portions are configured to provide stress relief for the cannula.

## Patentansprüche

1. Nadelvorrichtung (200) zur Gewebeentnahme, umfassend:
eine langgestreckte röhrenförmige Kanüle (204) mit einer Kanülenwand, die ein Kanülenlumen definiert;
worin sich das Kanülenlumen längs durch die Kanüle erstreckt;
wobei ein distales abgeschrägtes Ende (210) der Kanüle eine lange Seite (210a) und eine kurze Seite (210b) aufweist;
eine Kerbe (220) durch die Kanülenwand, die zum Kanülenlumen hin offen ist;
worin die Kerbe proximal neben dem abgeschrägten distalen Kanülenende angeordnet ist und allgemein in Längsausrichtung mit der langen abgeschrägten Endseite und gegenüber der kurzen abgeschrägten Endseite zentriert ist;
**dadurch gekennzeichnet, dass** die Kerbe eine distale Lippe (224) aufweist, die von einem Abschnitt der Kanülenwand definiert wird, wobei die distale Lippe ausgebildet ist, sich proximal von einem distalsten Ende der Kerbe zu erstrecken, so dass eine mittiger distaler Lippenabschnitt proximal von Lippenendabschnitten angeordnet ist, die mit allgemein längs verlaufenden lateralen Seiten der Kerbe fortlaufend sind; und
worin die distale Lippe eine proximal weisende Schneidkante (225) aufweist;
ferner umfassend ein Muster von echogenen Oberflächenmerkmalen (240), die unmittelbar neben und proximal von der Kerbe angeordnet sind.

2. Vorrichtung nach Anspruch 1, ferner umfassend einen Mandrin, der durch zumindest einen Längsabschnitt des Kanülenlumens angeordnet ist und im Wesentlichen einen gesamten Querschnittsbereich davon besetzt, worin ein distales Ende des Mandrins abgeschrägt ist, um sich mit dem distalen abgeschrägten Kanülenende im Wesentlichen auf planparallele Weise auszurichten.

3. Vorrichtung nach Anspruch 1, worin die Kanüle eine 19 Gauge Nadel mit einem Innendurchmesser von ungefähr 0,9 mm (ungefähr 0,037 Zoll) aufweist, worin die Kerbe ungefähr eine Hälfte eines Außenumfangs der Kanüle besetzt.

4. Vorrichtung nach Anspruch 3, worin eine umfangsmäßig mittige longitudinale Länge der Kerbe von dem proximalen Kerbenende zu dem mittigen distalen Lippenabschnitt ungefähr 4 mm (ungefähr 0,16 Zoll) beträgt.

5. Vorrichtung nach Anspruch 4, worin die mittige distale Lippe ungefähr 0,6 mm (ungefähr 0,025 Zoll) längs von einem distalsten Ende der Kerbe angeordnet ist.

6. Vorrichtung nach Anspruch 5, worin eine gekrümmte Übergangsfläche zwischen der distalen Lippe und den allgemein längs verlaufenden lateralen Seiten der Kerbe einen Krümmungsradius von ungefähr 0,05 mm (0,002 Zoll) umfasst.

7. Vorrichtung nach Anspruch 1, worin die echogenen Oberflächenmerkmale Vertiefungen in der Kanülenwand umfassen.

8. Vorrichtung nach Anspruch 1, worin die Kanüle zur Durchführung durch einen Arbeitskanal einer peroralen chirurgischen Visualisierungsvorrichtung ausreichend flexibel ausgebildet ist.

9. Vorrichtung nach Anspruch 1, worin die Kanüle eine Nadel, ausgewählt unter einer 19 Gauge Nadel, einer 22 Gauge Nadel, oder einer 25 Gauge Nadel, umfasst, worin die Kerbe ungefähr eine Hälfte eines Außenumfangs der Kanüle besetzt.

10. Vorrichtung nach Anspruch 1, worin die Lippenendabschnitte ausgebildet sind, Spannungsentlastung für die Kanüle bereitzustellen.

## Revendications

1. Dispositif d'aiguille d'échantillonnage de tissu (200), comprenant:
une canule tubulaire allongée (204) comprenant une paroi de canule définissant une lumière de canule,
dans lequel la lumière de canule s'étend de façon longitudinale à travers la canule,
une extrémité distale biseautée (210) de la canule comprenant un côté long (210a) et un côté court (210b),
une encoche (220) à travers la paroi de canule, ouverte vers la lumière de canule,
dans lequel l'encoche est disposée dans une direction proximale de façon adjacente à l'extrémité distale biseautée de la canule et est essentiellement centrée en alignement longitudinal avec le côté d'extrémité biseauté long et opposée au côté d'extrémité biseauté court,
**caractérisé en ce que** l'encoche comporte une lèvre distale (224) définie par une partie de la paroi de la canule, la lèvre distale étant configurée de manière à s'étendre dans une direction proximale à partir d'une extrémité distale extrême de l'encoche de telle sorte qu'une partie de lèvre distale centrale soit disposée à proximité des parties d'extrémité de lèvre qui sont continues aux côtés latéraux essentiellement longitudinaux de l'encoche et
dans lequel la lèvre distale comprend un bord de coupe orienté dans une direction proximale (225),
comprenant en outre un motif de caractéristiques de surface échogènes (240) disposé de façon immédiatement adjacente à et à proximité de l'encoche.

2. Dispositif selon la revendication 1, comprenant en outre un stylet disposé à travers et occupant sensiblement la totalité d'une surface de section transversale d'au moins une partie dans le sens de la longueur de la lumière de la canule, dans lequel une extrémité distale du stylet est biseautée de manière à s'aligner avec la canule biseautée distale d'une manière essentiellement coplanaire.

3. Dispositif selon la revendication 1, dans lequel la canule comprend une aiguille de calibre 19 présentant un diamètre intérieur d'environ 0,9 mm (environ 0,037 pouce), dans lequel l'encoche occupe environ une moitié d'une circonférence extérieure de la canule.

4. Dispositif selon la revendication 3, dans lequel une longueur longitudinale circonférentiellement centrale de l'encoche entre l'extrémité proximale de l'encoche proximale et la partie de lèvre distale centrale est d'environ 4 mm (environ 0,16 pouce).

5. Dispositif selon la revendication 4, dans lequel la lèvre distale centrale est disposée à environ 0,6 mm (environ 0,025 pouce) longitudinalement d'une extrémité distale extrême de l'encoche.

6. Dispositif selon la revendication 5, dans lequel une surface courbe de transition entre la lèvre distale et les côtés latéraux essentiellement longitudinaux de l'encoche présente un rayon de courbure d'environ 0,05 mm (0,002 pouce).

7. Dispositif selon la revendication 1, dans lequel les caractéristiques de surface échogènes comprennent des bosses dans la paroi de la canule.

8. Dispositif selon la revendication 1, dans lequel la canule est configurée de manière à être suffisamment flexible pour le passage à travers un canal de travail d'un dispositif de visualisation chirurgical per-oral.

9. Dispositif selon la revendication 1, dans lequel la canule comprend une aiguille sélectionnée parmi une aiguille de calibre 19, une aiguille de calibre 22 ou une aiguille de calibre 25, dans lequel l'encoche occupe environ la moitié d'une circonférence extérieure de la canule.

10. Dispositif selon la revendication 1, dans lequel les parties d'extrémité de lèvre sont configurées de manière à assurer un relâchement de contrainte pour la canule.
